# EUROPEAN PATENT APPLICATION

(11) **EP 4 155 387 A1**
(43) Date of publication of application: **29.03.2023**
(21) Application number: 21807713.9
(22) Date of filing: 17.05.2021
(51) Int. Cl.: C12N 5/077, C12N 5/0775, C12N 5/078, C12N 5/10

(54) **METHOD FOR PRODUCING TARGET CELLS, METHOD FOR PRODUCING PRODUCT USING TARGET CELLS, AND SERUM-FREE MEDIUM**

(30) Priority: 18.05.2020 JP 2020086836
(71) Applicant: Myoridge Co. Ltd., Kyoto-shi, Kyoto 606-8305 (JP)
(72) Inventor: MINAMI, Itsunari, Kyoto-shi, Kyoto 606-8305 (JP); MOTEGI, Itsuro, Kyoto-shi, Kyoto 613-0916 (JP); ETO, Koji, Kyoto-shi, Kyoto 606-8501 (JP)
(74) Representative: AWA Sweden AB
(86) International application number: PCT/JP2021/018542
(87) International publication number: WO 2021/235377

(57) **Abstract**

A method for producing a target cell includes producing a target cell by culturing a raw material cell in a serum-free medium containing: at least one culture factor selected from a growth factor and a differentiation inducing factor; human serum albumin; and at least one additive selected from a group consisting of a Src inhibitor, a PKC activator, methylcellulose, an Essential 8 medium, recombinant human serum albumin, and non-human animal serum albumin.

## Description

### FIELD

This patent application claims the benefit of priority from Japanese Patent Application No. 2020-086836 filed on May 18, 2020, the entire contents of which are incorporated herein by reference.

The present invention relates to a cell culture technique using a serum alternative.

### BACKGROUND

Conventionally, animal cells including human cells are cultured by further adding serum to a chemically defined medium. In addition to proteins such as albumin and globin, serum contains various components necessary for maintaining functions of a living body. For example, fetal bovine serum is used for culturing animal cells including human cells. Serum is derived from animals, which causes problems of cost, inter-lot differences, etc. In order to solve these problems, a serum-free medium containing a serum alternative instead of serum has been developed (for example, see Patent Literature 1).

### CITATION LIST

### PATENT LITERATURE

PATENT LITERATURE 1: WO2014/144198

### SUMMARY

### TECHNICAL PROBLEM

As described above, serum is derived from animals and thus involves problems of quality stability of cultured cells due to inter-lot differences and problems of cost, especially in the case of culturing a large number of cells. On the other hand, there is a concern that a serum alternative may lower the culture efficiency of cells as compared to serum. Therefore, the present invention aims to provide a cell culture technique using a serum alternative, which can maintain a culture efficiency of cells comparable to that in the use of serum, and which can also solve the problems of cost and quality stability of cultured cells otherwise accompanying the use of serum.

### SOLUTION TO PROBLEM

According to one aspect, there is provided a method for producing a target cell, the method comprising producing the target cell by culturing a raw material cell in a serum-free medium containing:
at least one culture factor selected from a growth factor and a differentiation inducing factor;
human serum albumin; and
at least one additive selected from a group consisting of a Src inhibitor, a PKC activator, methylcellulose, an Essential 8 medium, recombinant human serum albumin, and non-human animal serum albumin.

According to another aspect, there is provided a method for producing a product using target cells, the method including comprising producing target cells and producing a product using the target cells by culturing raw material cells according to the method described above.

According to still another aspect, there is provided a serum-free medium for use in production of target cells, the serum-free medium containing: at least one culture factor selected from a growth factor and a differentiation inducing factor; human serum albumin; and at least one additive selected from a group consisting of a Src inhibitor, a PKC activator, methylcellulose, an Essential 8 medium, recombinant human serum albumin, and non-human animal serum albumin.

There is provided a serum-free medium for use in production of a product using target cells, the serum-free medium containing: at least one culture factor selected from a growth factor and a differentiation inducing factor; human serum albumin; and at least one additive selected from a group consisting of a Src inhibitor, a PKC activator, methylcellulose, an Essential 8 medium, recombinant human serum albumin, and non-human animal serum albumin.

### ADVANTAGEOUS EFFECTS OF INVENTION

According to the present invention, it is possible to provide a cell culture technique using a serum alternative, which can maintain the culture efficiency of cells obtained when serum is used, and which can also solve the problems of cost and quality stability of cultured cells that occur when serum is used.

### BRIEF DESCRIPTION OF THE DRAWINGS

FIG. 1 is a graph showing the number of megakaryocytes differentiated in respective media.
FIG. 2 is a graph showing the number of megakaryocytes differentiated in respective media.
FIG. 3 is a graph showing the number of megakaryocytes differentiated in respective media.
FIG. 4A is a microscopic image showing megakaryocytes stained with a nuclear staining reagent.
FIG. 4B is a microscopic image showing megakaryocytes stained with a nuclear staining reagent.
FIG. 4C is a microscopic image showing megakaryocytes stained with a nuclear staining reagent.
FIG. 4D is a microscopic image showing megakaryocytes stained with a nuclear staining reagent.
FIG. 4E is a microscopic image showing megakaryocytes stained with a nuclear staining reagent.
FIG. 5 is a graph showing the ratio of cells positive for thrombocyte markers (CD41 and CD42b).
FIG. 6 is a graph showing the ratio of cells positive for a thrombocyte marker (PAC1).
FIG. 7 is a graph showing the number of iPS cells grown in respective media.
FIG. 8 is a graph showing the number of mesenchymal stem cells grown in respective media.

### DETAILED DESCRIPTION

Hereinafter, the present invention will be described in detail; however, the following description is intended to explicate the present invention and is not intended to limit the present invention.

The inventors of the present invention carried out screening for a serum alternative among various substances. As a result, the inventors have found that when a combination of (a) human serum albumin and (b) at least one additive selected from a group consisting of a Src inhibitor, a PKC activator, methylcellulose, an Essential 8 medium, recombinant human serum albumin, and non-human animal serum albumin is used as a serum alternative, the culture efficiency of cells obtained when serum is used can be maintained, and thus have accomplished the present invention. In this description, the term "culture efficiency" refers to the differentiation efficiency in the case of a differentiation culture and the proliferation efficiency in the case of a proliferation culture.

### <1. Method of Producing Target Cells>

According to one aspect, there is provided a method for producing target cells, the method comprising producing the target cells by culturing raw material cells in a serum-free medium containing: at least one culture factor selected from a growth factor and a differentiation inducing factor; human serum albumin; and at least one additive selected from a group consisting of a Src inhibitor, a PKC activator, methylcellulose, an Essential 8 medium, recombinant human serum albumin, and non-human animal serum albumin.

In one embodiment, the culture factor is at least one differentiation inducing factor and the target cells are differentiated cells. A differentiation inducing factor has an action of promoting differentiation of the raw material cells. In this embodiment, the aforementioned method is a "method for producing differentiated cells". That is, the "method for producing differentiated cells" includes producing differentiated cells by culturing raw material cells in a serum-free medium containing: at least one differentiation inducing factor; human serum albumin; and at least one additive selected from a group consisting of a Src inhibitor, a PKC activator, methylcellulose, an Essential 8 medium, recombinant human serum albumin, and non-human animal serum albumin.

In another embodiment, the culture factor is at least one growth factor and the target cells are proliferating cells. The growth factor has an action of promoting the proliferation of raw material cells. In this embodiment, the aforementioned method is a "method for producing proliferating cells". That is, the "method for producing proliferating cells" includes producing proliferating cells by culturing raw material cells in a serum-free medium containing: at least one growth factor; human serum albumin; and at least one additive selected from a group consisting of a Src inhibitor, a PKC activator, methylcellulose, an Essential 8 medium, recombinant human serum albumin, and non-human animal serum albumin.

Hereinafter, the "method for producing differentiated cells" and the "method for producing proliferating cells" will be concretely described.

### <1-1. Method for Producing Differentiated Cells>

### (Cells)

A method for producing differentiated cells produces differentiated cells by culturing raw material cells in the presence of a differentiation inducing factor.

The raw material cells may be selected from a group consisting of, for example, megakaryocyte progenitor cells, iPS cells, mesenchymal stem cells, and fibroblasts. The raw material cells may preferably be selected from a group consisting of human megakaryocyte progenitor cells, human iPS cells, human mesenchymal stem cells, and human fibroblasts.

The differentiated cells may be selected from a group consisting of, for example, megakaryocytes, mesenchymal stem cells, fibroblasts, and cardiomyocytes. The differentiated cells may preferably be selected from a group consisting of human megakaryocytes, human mesenchymal stem cells, human fibroblasts, and human cardiomyocytes.

In one example, the raw material cells are megakaryocyte progenitor cells and the differentiated cells are megakaryocytes. Preferably, the raw material cells are human megakaryocyte progenitor cells and the differentiated cells are human megakaryocytes.

In another example, the raw material cells are iPS cells and the differentiated cells are mesenchymal stem cells. Preferably, the raw material cells are human iPS cells, and the differentiated cells are human mesenchymal stem cells.

In yet another embodiment, the raw material cells are iPS cells and the differentiated cells are fibroblasts. Preferably, the raw material cells are human iPS cells and the differentiated cells are human fibroblasts.

In yet another example, the raw material cells are iPS cells and the differentiated cells are cardiomyocytes. Preferably, the raw material cells are human iPS cells and the differentiated cells are human cardiomyocytes.

In yet another embodiment, the raw material cells are mesenchymal stem cells and the differentiated cells are cardiomyocytes. Preferably, the raw material cells are human mesenchymal stem cells and the differentiated cells are human cardiomyocytes.

In yet another embodiment, the raw material cells are fibroblasts and the differentiated cells are cardiomyocytes. Preferably, the raw material cells are human fibroblasts and the differentiated cells are human cardiomyocytes.

### (Serum Alternative)

The method for producing differentiated cells is characterized by using, as a serum alternative, a combination of:
(a) human serum albumin; and
(b) at least one additive selected from a group consisting of a Src inhibitor, a PKC activator, methylcellulose, an Essential 8 medium, recombinant human serum albumin, and non-human animal serum albumin.

Human serum albumin is prepared from human plasma or serum, and is not recombinant. Human serum albumin is commercially available from, for example, Sigma-Aldrich.

The additive is preferably a Src inhibitor. That is, the serum alternative is preferably a combination of human serum albumin and a Src inhibitor. The term "Src inhibitor" refers to a substance that inhibits a tyrosine kinase Src or its downstream signaling pathway. As the Src inhibitor, a known Src inhibitor can be used. Examples of the Src inhibitor include A419259, SU6656, PP1, 1-naphthyl PP1, PP2, indirubin-3'-(2,3-dihydroxypropyl)-oxime ether), TX-1123, Src kinase inhibitor I (CAS 179248-59-0), AZM475271, bosutinibh, herbimycin A, KB SRC4, MNS, PD166285, TC-S7003, etc.

The Src inhibitor is preferably A419259. A419259 has the following structure, and the compound name of A419259 is 7-[4-(4-methylpiperazin-1-yl)cyclohexyl]-5-(4-phenoxyphenyl)pyrrolo[2,3-d]pyrimidin-4-amine.

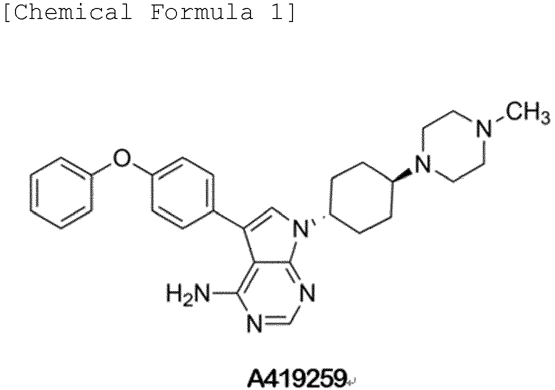

A419259 may be added in the form of salt. A419259 is commercially available from, for example, FUJIFILM Wako Pure Chemical Corporation.

As another option, the additive is preferably a PKC activator. That is, the serum alternative is preferably a combination of human serum albumin and a PKC activator. The term "PKC activator" refers to a substance that activates protein kinase C (PKC) or its downstream signaling pathway. As the PKC activator, a known PKC activator can be used. Examples of the PKC activator include phorbol 12-myristate 13-acetate (PMA), prostratin, bryostatin 1, bryostatin 2, FR236924, (-)-Indolactam V, PEP005, phorbol 12,13-dibutyrate, SC-9, SC-10, 1-Oleoyl-2-acetyl-sn-glycerol, 1-O-Hexadecyl-2-O-arachidonyl-sn-glycerol, 1,2-Dioctanoyl-sn-glycerol, PIP2, Resiniferatoxin, Phorbol 12,13-Dihexanoate, Mezerein, Ingenol 3-Angelate, RHC-80267, DCP-LA, Lipoxin A4, etc.

The PKC activator is preferably prostratin. The prostratin has the following structure, and the compound name of prostratin is (1aR, 1bS, 4aR, 7aS, 7bR, 8R, 9aS)-4a, 7b-dihydroxy-3-(hydroxymethyl)-1,1,6,8-tetramethyl-5-oxo-1, 1a, 1b, 4, 4a, 5, 7a, 7b, 8, 9-decahydro-9aH-cyclopropa[3,4]benz[1,2-e]azulen-9a-yl acetate. The prostratin is commercially available from, for example, FUJIFILM Wako Pure Chemical Corporation.

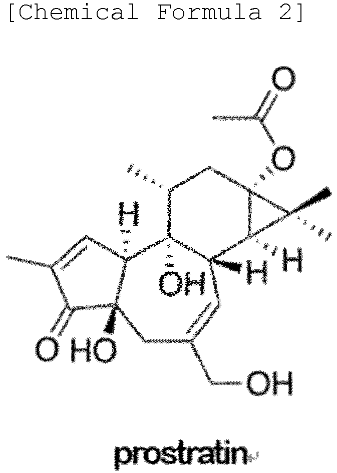

As yet another option, the additive is preferably methylcellulose. That is, the serum alternative is preferably a combination of human serum albumin and methylcellulose. The methylcellulose is commercially available from, for example, R&D Systems.

As yet another option, the additive is preferably an Essential 8 medium. That is, the serum alternative is preferably a combination of human serum albumin and an Essential 8 medium. According to Article 1 (Chen G et al, Chemically defined conditions for human iPSC derivation and culture. Nat Methods. 2011 May; 8(5) :424-9. doi: 10.1038/nmeth.1593. Epub 2011 Apr 10.), the Essential 8 medium has the following composition: DMEM/F12, L-ascorbic acid-2-phosphate magnesium (64 mg/l), sodium selenium (14 µg/l), FGF2 (100 µg/l), insulin (19.4 mg/l), NaHCO₃ (543 mg/l), transferrin (10.7 mg/l), and either TGFβ1 (2 µg/l) or NODAL (100 pg/l). The Essential 8 medium is commercially available from, for example, Invitrogen.

As yet another option, the additive is preferably recombinant human serum albumin or non-human animal serum albumin. That is, the serum alternative is preferably a combination of human serum albumin and either recombinant human serum albumin or non-human animal serum albumin. The recombinant human serum albumin is, for example, human serum albumin expressed using oryza sativa or human serum albumin expressed using yeast. The non-human animal serum albumin is, for example, bovine serum albumin. The recombinant human serum albumin and non-human animal serum albumin are commercially available from, for example, Sigma-Aldrich or FUJIFILM Wako Pure Chemical Corporation.

The serum alternative can be used at the concentrations described below. The concentrations described below represent the final concentration in the culture medium.

The concentration of human serum albumin in a serum-free medium is, for example, 0.01 to 2.0 mass%, preferably 0.05 to 1.5 mass%, and more preferably 0.075 to 0.75 mass%.

In the case of a Src inhibitor such as A419259 being used as the additive, the concentration of Src inhibitor in the serum-free medium is, for example, 0.001 to 10 µM, preferably 0.01 to 1.0 µM, and more preferably 0.03 to 0.2 µM.

In the case of a PKC activator such as prostratin being used as the additive, the concentration of the PKC activator in the serum-free medium is, for example, 0.001 to 10 µM, preferably 0.01 to 1.0 µM, and more preferably 0.015 to 0.2 µM.

In the case of methylcellulose being used as the additive, the concentration of methylcellulose in the serum-free medium is, for example, 0.001 to 3.0 mass%, preferably 0.01 to 1.0 mass%, and more preferably 0.018 to 0.3 mass%.

In the case of an Essential 8 medium being used as the additive, the concentration of the Essential 8 medium in the serum-free medium is, for example, 0.5 to 100 mass%, preferably 0.5 to 50 mass%, and more preferably 0.625 to 30 mass%.

In the case of recombinant human serum albumin being used as the additive, the concentration of recombinant human serum albumin in the serum-free medium is, for example, 0.001 to 1.0 mass%, preferably 0.01 to 0.5 mass%, and more preferably 0.015 to 0.3 mass%.

In the case of non-human animal serum albumin being used as the additive, the concentration of non-human animal serum albumin in the serum-free medium is, for example, 0.01 to 2.0 mass%, preferably 0.05 to 1.5 mass%, and more preferably 0.125 to 0.75 mass%.

### (Culture)

The method for producing differentiated cells can be carried out according to a method similar to the method for a conventional differentiation culture using serum, except that a combination of (a) human serum albumin and (b) at least one additive selected from a group consisting of a Src inhibitor, a PKC activator, methylcellulose, an Essential 8 medium, recombinant human serum albumin, and non-human animal serum albumin is used as the serum alternative. To be more specific, the method for producing differentiated cells can be carried out by culturing raw material cells in a serum-free medium containing a basal medium (for example, MEM, DMEM, IMDM, Ham's F-12, DMEM/F12, RPMI1640, etc.), a differentiation inducing factor, and the aforementioned serum alternative.

As the differentiation inducing factor, a known factor can be used depending on the types of target cells and differentiated cells. The differentiation inducing factor may have an action of promoting proliferation of raw material cells in addition to an action of promoting differentiation thereof. In addition, the differentiation culture may be composed of a single culture step using one or more specific differentiation inducing factors, or may be composed of a plurality of culture steps using one or more different differentiation inducing factors for each culture step.

By culturing, the raw material cells are differentiated to produce differentiated cells. However, during the culture, proliferation of cells may occur in addition to differentiation thereof.

For example, a differentiation culture from megakaryocyte progenitor cells to megakaryocytes can be carried out by adding a megakaryocyte differentiation inducing factor and the above-described serum alternative to a basal medium to prepare a serum-free medium and then culturing the megakaryocyte progenitor cells in this serum-free medium. As the basal medium, for example, IMDM can be used, and as the megakaryocyte differentiation inducing factor, for example, SCF, TA-316, KP-457, GNF-351, and Y39983 can be used.

SCF is a known protein called a stem cell factor and is available from, for example, R&D Systems. TA-316 has the following structure and is available from, for example, Nissan Chemical Corporation. KP-457 has the following structure and is available from, for example, GLPBIO (https://www.glpbio.com/kp-457.html). GNF-351 has the following structure and is available from, for example, Santa Cruz Biotechnology (https://www.scbt.com/ja/p/ahr-antagonist-iii-gnf351). Y39983 has the following structure and is available from, for example, Selleck Biotechnology Co., Ltd (https://www.selleck.co.jp/products/y-39983-hcl.html) .

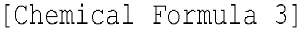

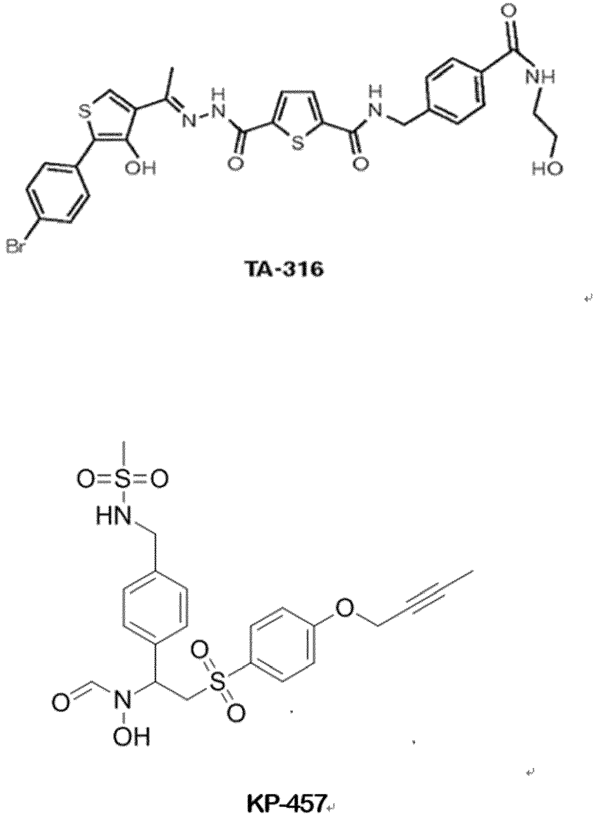

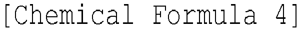

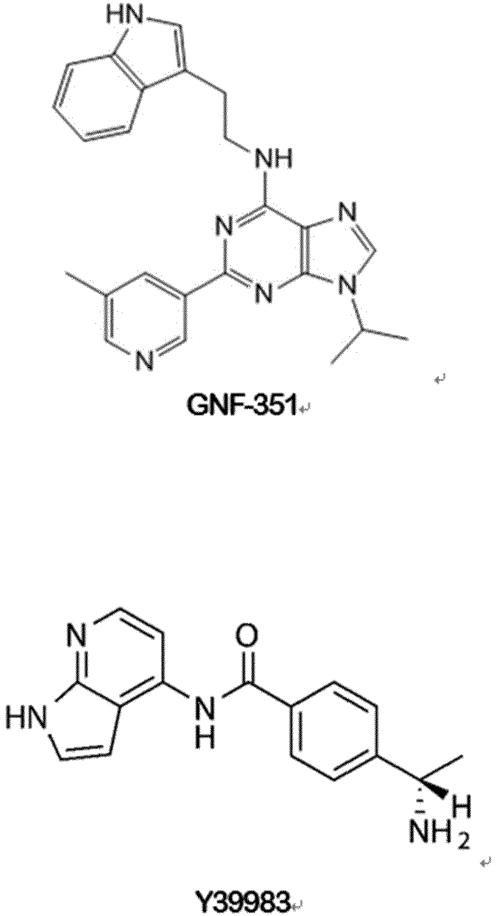

KP-457 is known to function as a material for retaining the functions of platelets produced using megakaryocytes, i.e., as a platelet function-retaining agent (see WO2012/157586). In addition, GNF-351 and Y-39983 are known to function as a substance that promotes the production of platelets using megakaryocytes, i.e., as a platelet-production promoter (see WO2016/204256).

The megakaryocyte differentiation inducing factor was described as an example in the above description; however, it is clear that the differentiation inducing factor is contained in the serum-free medium in the case where the differentiation of the cells is caused by carrying out the "method for producing differentiated cells" described above, and it is not always necessary to specify which component in the serum-free medium is the differentiation inducing factor.

### (Effect)

The method described above is superior in that the differentiation efficiency of cells obtained when serum is used can be maintained even though the method uses the serum alternative. In addition, according to the method described above, use of the serum alternative can solve the problems of cost and quality stability of cultured cells that occur when serum is used.

### <1-2. Method for Producing Proliferating Cells>

The method for producing proliferating cells produces proliferating cells by culturing raw material cells in the presence of a growth factor. In this description, the term "growth factor" refers to any substance that contributes to the proliferation of cells, such as a substance serving as a nutrient for the proliferation of cells or a substance that promotes the proliferation of cells.

The raw material cells and the target cells may be selected from, for example, the group consisting of iPS cells and mesenchymal stem cells. The raw material cells and the target cells may preferably be selected from a group consisting of human iPS cells and human mesenchymal stem cells.

The method for producing proliferating cells is characterized by using, as a serum alternative, a combination of (a) human serum albumin and (b) at least one additive selected from a group consisting of a Src inhibitor, a PKC activator, methylcellulose, an Essential 8 medium, recombinant human serum albumin, and non-human animal serum albumin.

The serum alternative is as described in the above section <1-1. Method for Producing Differentiated Cells>. The serum alternative can be used at the concentration described in the above section <1-1. Method for Producing Differentiated Cells>.

The method for producing proliferating cells can be carried out according to a method similar to the method for a conventional proliferation culture using serum, except that a combination of (a) human serum albumin and (b) at least one additive selected from a group consisting of a Src inhibitor, a PKC activator, methylcellulose, an Essential 8 medium, recombinant human serum albumin, and non-human animal serum albumin is used as the serum alternative. To be more specific, the method for producing proliferating cells can be carried out by culturing raw material cells in a serum-free medium containing a basal medium (for example, MEM, DMEM, IMDM, Ham's F-12, DMEM/F12, RPMI1640, etc.) containing a growth factor, and the aforementioned serum alternative.

The basal medium usually contains a growth factor such as an amino acid, vitamin, inorganic salt, etc. However, in addition to the growth factor contained in the basal medium, an additional growth factor may be added to the basal medium.

For example, a proliferation culture of iPS cells can be performed by adding the aforementioned serum alternative to a commercially available medium for proliferation of iPS cells to prepare a serum-free medium, thereby culturing the iPS cells in this serum-free medium. For example, DMEM/F12 can be used as a medium for proliferation of iPS cells, and DMEM/F12 contains, for example, L-glutamine, vitamin B12, etc., as an iPS cell growth factor. As the serum alternative, preferably a combination of human serum albumin and prostratin or a combination of human serum albumin and recombinant human serum albumin can be used.

For example, a proliferation culture of mesenchymal stem cells can be performed by adding the aforementioned serum alternative to a commercially available medium for proliferation of mesenchymal stem cells to prepare a serum-free medium, thereby culturing mesenchymal stem cells in this serum-free medium. For example, DMEM/F12 can be used as a medium for proliferation of mesenchymal stem cells, and DMEM/F12 contains, for example, L-glutamine, vitamin B12, etc., as a mesenchymal stem cell growth factor. As the serum alternative, preferably, a combination of human serum albumin and A419259 or a combination of human serum albumin and recombinant human serum albumin can be used.

As another option, it is also possible to perform a proliferation culture on megakaryocyte progenitor cells by applying the above-described method to the megakaryocyte progenitor cells, or to perform a proliferation culture on cardiomyocytes by applying the above-described method to the cardiomyocytes.

The iPS cell growth factor and the mesenchymal stem cell growth factor were described as an example in the above description; however, it is clear that the growth factor is contained in the serum-free medium in the case where the proliferation of cells is caused by carrying out the "method for producing proliferating cells", and it is not always necessary to specify which component in the serum-free medium is the growth factor.

The method described above is superior in that the proliferation efficiency of cells obtained when serum is used can be maintained even though the method uses the serum alternative. In addition, according to the method described above, use of the serum alternative can solve the problems of cost and quality stability of cultured cells that occur when serum is used.

### <2. Method for Producing Product Using Target Cells>

According to another aspect, there is provided a method for producing a product using target cells, the method comprising producing target cells and producing a product using the target cells by culturing raw material cells according to the method described above. That is, there is provided a method for producing a product using target cells, the method comprising producing target cells and producing a product using the target cells by culturing raw material cells in a serum-free medium containing: at least one culture factor selected from a growth factor and a differentiation inducing factor; human serum albumin; and at least one additive selected from a group consisting of a Src inhibitor, a PKC activator, methylcellulose, an Essential 8 medium, recombinant human serum albumin, and non-human animal serum albumin.

By this method, a culture of the raw material cells can be performed according to the same method as that described in the above section <1-1. Method for Producing Differentiated Cells>. The method may further contain a step of collecting the product produced using the target cells.

According to one embodiment, in a method for producing a product using target cells, the raw material cells are megakaryocyte progenitor cells, the target cells are megakaryocytes, and the product is platelets. That is, according to one aspect, there is provided a method for producing platelets using megakaryocytes, the method comprising producing megakaryocytes and producing the platelets using the megakaryocytes by culturing megakaryocyte progenitor cells in a serum-free medium containing: at least one megakaryocyte differentiation inducing factor; human serum albumin; and at least one additive selected from a group consisting of a Src inhibitor, a PKC activator, methylcellulose, an Essential 8 medium, recombinant human serum albumin, and non-human animal serum albumin.

For example, production of platelets using megakaryocytes can be performed by adding a megakaryocyte differentiation inducing factor and the aforementioned serum alternative to a basal medium to prepare a serum-free medium and then culturing the megakaryocyte progenitor cells in this serum-free medium. As the basal medium, for example, IMDM can be used, and as the megakaryocyte differentiation inducing factor, for example, SCF, TA-316, KP-457, GNF-351, and Y39983 can be used. By the culturing, the megakaryocyte progenitor cells are differentiated to produce megakaryocytes, and platelets are produced using the produced megakaryocytes.

As described above, KP-457 is known to function as a material for retaining the functions of platelets produced using megakaryocytes, i.e., as a platelet function-retaining agent (see WO2012/157586). In addition, GNF-351 and Y-39983 are known to function as a substance that promotes the production of platelets using megakaryocytes, i.e., as a platelet-production promoter (see WO2016/204256).

Alternatively, another embodiment may enable iPS cells or mesenchymal stem cells to produce cytokines by performing a proliferation culture thereon.

That is, according to another aspect, there is provided a method for producing cytokines using iPS cells, the method comprising producing cytokines by culturing the iPS cells in a serum-free medium containing: at least one iPS cell growth factor; human serum albumin; and at least one additive selected from a group consisting of a Src inhibitor, a PKC activator, methylcellulose, an Essential 8 medium, recombinant human serum albumin, and non-human animal serum albumin.

By this method, a culture of iPS cells can be performed according to the same method as that described in connection with the proliferation culture on the iPS cells in the above section <1-2. Method for Producing Proliferating Cells>. As a result of the culture, iPS cells proliferate and cytokines are produced using the iPS cells.

According to still another aspect, there is provided a method for producing cytokines using mesenchymal stem cells, the method comprising producing cytokines by culturing mesenchymal stem cells in a serum-free medium containing: at least one mesenchymal stem cell growth factor; human serum albumin; and at least one additive selected from a group consisting of a Src inhibitor, a PKC activator, methylcellulose, an Essential 8 medium, recombinant human serum albumin, and non-human animal serum albumin.

By this method, a culture of mesenchymal stem cells can be performed according to the same method as that described in connection with the proliferation culture on the mesenchymal stem cells in the above section <1-2. Method for Producing Proliferating Cells>. By culturing, mesenchymal stem cells proliferate, and cytokines are produced using the mesenchymal stem cells.

The method described above is superior in that the culture efficiency of cells and the production efficiency of a product using the cells obtained when serum is used can be maintained even though the method uses the serum alternative. In addition, according to the method described above, use of the serum alternative can solve the problems of cost and quality stability of cultured cells obtained when serum is used.

### <3. Serum-free Medium>

### <3-1. Serum-free Medium for Use in Production of Target Cells>

According to another aspect, there is provided a serum-free medium for use in production of target cells, the serum-free medium containing: at least one culture factor selected from a growth factor and a differentiation inducing factor; human serum albumin; and at least one additive selected from a group consisting of a Src inhibitor, a PKC activator, methylcellulose, an Essential 8 medium, recombinant human serum albumin, and non-human animal serum albumin.

This serum-free medium is used in the production of target cells, and specifically, can be used for the method described in the above section <1. Method of Producing Target Cells>. That is, the serum-free medium can be used in the production of proliferating cells or in the production of differentiated cells. Therefore, for the details of the serum-free medium, reference can be made to the description of the medium described in the above section <1. Method of Producing Target Cells>.

The serum-free medium is characterized in including, as a serum alternative, a combination of (a) human serum albumin and (b) at least one additive selected from a group consisting of a Src inhibitor, a PKC activator, methylcellulose, an Essential 8 medium, recombinant human serum albumin, and non-human animal serum albumin. The serum alternative is as described in the above section <1-1. Method for Producing Differentiated Cells>. In addition, the serum alternative may be contained in the serum-free medium at the concentration described in the above section <1-1. Method for Producing Differentiated Cells>.

In one embodiment, the serum-free medium is a medium obtained by replacing the serum contained in a known cell differentiation-inducing medium containing a differentiation inducing factor with the serum alternative described above. In this embodiment, the serum-free medium contains a basal medium (e.g., MEM, DMEM, IMDM, Ham's F-12, DMEM/F12, RPMI1640, etc.), a differentiation inducing factor, and the serum alternative described above.

In another embodiment, the serum-free medium is a medium obtained by replacing the serum contained in a known medium for proliferation of cells containing a growth factor with the serum alternative described above. In this embodiment, the serum-free medium contains a basal medium (e.g., MEM, DMEM, IMDM, Ham's F-12, DMEM/F12, RPMI1640, etc.) containing a growth factor, and the serum alternative described above.

According to a specific embodiment, in a serum-free medium for use in production of target cells, the target cells are megakaryocytes and the culture factor is a megakaryocyte differentiation inducing factor. That is, in this specific embodiment, the serum-free medium comprises a basal medium (such as IMDM), a megakaryocyte differentiation inducing factor, and the serum alternative described above. As described above, examples of the megakaryocyte differentiation inducing factor include SCF, TA-316, KP-457, GNF-351, and Y39983.

The serum-free medium described above is superior in that the culture efficiency (specifically, the differentiation efficiency and proliferation efficiency) of cells as in the use of a corresponding medium containing serum can be maintained even though the serum-free medium contains the serum alternative. In addition, according to the method described above, use of the serum alternative can solve the problems of cost and quality stability of cultured cells that occur when serum is used.

### <3-2. Serum-free Medium for Use in Production of Product Using Target Cells>

According to another aspect, there is provided a serum-free medium for use in production of a product using target cells, the serum-free medium containing: at least one culture factor selected from a growth factor and a differentiation inducing factor; human serum albumin; and at least one additive selected from a group consisting of a Src inhibitor, a PKC activator, methylcellulose, an Essential 8 medium, recombinant human serum albumin, and non-human animal serum albumin.

This serum-free medium is used in the production of a product using target cells, and specifically, can be used for the method described in the above section <2. Method of Producing Product Using Target Cells>. Therefore, for the details of the serum-free medium, reference can be made to the description of the medium described in the above section

### <2. Method of Producing Product Using Target Cells>.

The serum-free medium is characterized in including, as a serum alternative, a combination of (a) human serum albumin and (b) at least one additive selected from a group consisting of a Src inhibitor, a PKC activator, methylcellulose, an Essential 8 medium, recombinant human serum albumin, and non-human animal serum albumin. The serum alternative is as described in the above section <1-1. Method for Producing Differentiated Cells>. In addition, the serum alternative may be contained in the serum-free medium at the concentration described in the above section <1-1. Method for Producing Differentiated Cells>.

According to a specific embodiment, in a serum-free medium for use in production of a product using target cells, production of the product using the target cells is production of megakaryocytes and the culture factor is a megakaryocyte differentiation inducing factor. That is, in this specific embodiment, the serum-free medium comprises a basal medium (such as IMDM), a megakaryocyte differentiation inducing factor, and the serum alternative described above. As described above, examples of the megakaryocyte differentiation inducing factor include SCF, TA-316, KP-457, GNF-351, and Y39983.

As described above, KP-457 is known to function as a material for retaining the functions of platelets produced using megakaryocytes, i.e., as a platelet function-retaining agent (see WO2012/157586). In addition, GNF-351 and Y-39983 are known to function as a substance that promotes the production of platelets using megakaryocytes, i.e., as a platelet-production promoter (see WO2016/204256).

The serum-free medium described above is superior in that the culture efficiency of cells and the production efficiency of a product using the cells as in the use of a corresponding medium containing serum can be maintained even though the serum-free medium contains the serum alternative. In addition, according to the method described above, use of the serum alternative can solve the problems of cost and quality stability of cultured cells that occur when serum is used.

### <4. Preferred Embodiment>

Hereinafter, preferred embodiments of the present invention will be summarized.

### [A1]

A method for producing target cells, the method comprising producing the target cells by culturing raw material cells in a serum-free medium containing: at least one culture factor selected from a growth factor and a differentiation inducing factor; human serum albumin; and at least one additive selected from a group consisting of a Src inhibitor, a PKC activator, methylcellulose, an Essential 8 medium, recombinant human serum albumin, and non-human animal serum albumin.

### [A2]

The method according to [A1], wherein the culture factor is at least one differentiation inducing factor and the target cells are differentiated cells.

### [A3]

The method according to [A2], wherein the raw material cells are selected from a group consisting of megakaryocyte progenitor cells, iPS cells, mesenchymal stem cells, and fibroblasts.

### [A4]

The method according to [A2] or [A3], wherein the raw material cells are selected from a group consisting of human megakaryocyte progenitor cells, human iPS cells, human mesenchymal stem cells, and human fibroblasts.

### [A5]

The method according to any one of [A2] to [A4], wherein the target cells are selected from a group consisting of megakaryocytes, mesenchymal stem cells, fibroblasts, and cardiomyocytes.

### [A6]

The method according to any one of [A2] to [A5], wherein the target cells are selected from a group consisting of human megakaryocytes, human mesenchymal stem cells, human fibroblasts, and human cardiomyocytes.

### [A7]

The method according to any one of [A2], [A3], and [A5], wherein the raw material cells are megakaryocyte progenitor cells and the target cells are megakaryocytes.

### [A8]

The method according to any one of [A2] to [A7], wherein the raw material cells are human megakaryocyte progenitor cells and the target cells are human megakaryocytes.

### [A9]

The method according to any one of [A2], [A3], and [A5], wherein the raw material cells are iPS cells and the target cells are mesenchymal stem cells.

### [A10]

The method according to any one of [A2] to [A6] and [A9], wherein the raw material cells are human iPS cells and the target cells are human mesenchymal stem cells.

### [A11]

The method according to any one of [A2], [A3], and [A5], wherein the raw material cells are iPS cells and the target cells are fibroblasts.

### [A12]

The method according to any one of [A2] to [A6] and [A11], wherein the raw material cells are human iPS cells and the target cells are human fibroblasts.

### [A13]

The method according to any one of [A2], [A3], and [A5], wherein the raw material cells are iPS cells and the target cells are cardiomyocytes.

### [A14]

The method according to any one of [A2] to [A6] and [A13], wherein the raw material cells are human iPS cells and the target cells are human cardiomyocytes.

### [A15]

The method according to any one of [A2], [A3], and [A5], wherein the raw material cells are mesenchymal stem cells and the target cells are cardiomyocytes.

### [A16]

The method according to any one of [A2] to [A6] and [A15], wherein the raw material cells are human mesenchymal stem cells and the target cells are human cardiomyocytes.

### [A17]

The method according to any one of [A2], [A3] and [A5], wherein the raw material cells are fibroblasts and the target cells are cardiomyocytes.

### [A18]

The method according to any one of [A2] to [A6] and [A17], wherein the raw material cells are human fibroblasts and the target cells are human cardiomyocytes.

### [B1]

The method according to [A1], wherein the culture factor is at least one growth factor and the target cells are proliferating cells.

### [B2]

The method according to [B1], wherein the raw material cells and the target cells are selected from a group consisting of iPS cells and mesenchymal stem cells.

### [B3]

The method according to [B1] or [B2], wherein the raw material cells and the target cells are selected from a group consisting of human iPS cells and human mesenchymal stem cells.

### [B4]

The method according to [B1] or [B2], wherein the raw material cells and the target cells are iPS cells.

### [B5]

The method according to any one of [B1] to [B4], wherein the raw material cells and the target cells are human iPS cells.

### [B6]

The method according to [B1] or [B2], wherein the raw material cells and the target cells are mesenchymal stem cells.

### [B7]

The method according to any one of [B1] to [B3] and [B6], wherein the raw material cells and the target cells are human mesenchymal stem cells.

### [B8]

The method according to [B1], wherein the raw material cells and the target cells are megakaryocyte progenitor cells.

### [B9]

The method according to [B1] or [B8], wherein the raw material cells and the target cells are human megakaryocyte progenitor cells.

### [B10]

The method according to [B1], wherein the raw material cells and the target cells are cardiomyocytes.

### [B11]

The method according to [B1] or [B10], wherein the raw material cells and the target cells are human cardiomyocytes.

### [C1]

The method according to any one of [A1] to [A18] and [B1] to [B11], wherein the additive is a Src inhibitor, preferably A419259.

### [C2]

The method according to any one of [A1] to [A18] and [B1] to [B11], wherein the additive is a PKC activator, preferably prostratin.

### [C3]

The method according to any one of [A1] to [A18] and [B1] to [B11], wherein the additive is methylcellulose.

### [C4]

The method according to any one of [A1] to [A18] and [B1] to [B11], wherein the additive is an Essential 8 medium.

### [C5]

The method according to any one of [A1] to [A18] and [B1] to [B11], wherein the additive is recombinant human serum albumin or non-human animal serum albumin.

### [C6]

The method according to any one of [A1] to [A18], [B1] to [B11], and [C5], wherein the additive is recombinant human serum albumin, preferably recombinant human serum albumin expressed using oryza sativa or recombinant human serum albumin expressed using yeast.

### [C7]

The method according to any one of [A1] to [A18], [B1] to [B11], and [C5], wherein the additive is non-human animal serum albumin, preferably bovine serum albumin.

### [C8]

The method according to any one of [A1] to [A18], [B1] to [B11], [C1], to [C7], wherein the concentration (final concentration) of the human serum albumin in the serum-free medium is 0.01 to 2.0 mass%, preferably 0.05 to 1.5 mass%, and more preferably 0.075 to 0.75 mass%.

### [C9]

The method according to any one of [A1] to [A18], [B1] to [B11], [C1], and [C8], wherein the additive is a Src inhibitor, preferably A419259, and the concentration (final concentration) of the Src inhibitor in the serum-free medium is 0.001 to 10 µM, preferably 0.01 to 1.0 µM, and more preferably 0.03 to 0.2 µM.

### [C10]

The method according to any one of [A1] to [A18], [B1] to [B11], [C2], and [C8], wherein the additive is a PKC activator, preferably prostratin, and the concentration (final concentration) of the PKC activator in the serum-free medium is 0.001 to 10 µM, preferably 0.01 to 1.0 µM, and more preferably 0.015 to 0.2 µM.

### [C11]

The method according to any one of [A1] to [A18], [B1] to [B11], [C3], and [C8], wherein the additive is methylcellulose, and the concentration (final concentration) of the methylcellulose in the serum-free medium is 0.001 to 3.0 mass%, preferably 0.01 to 1.0 mass%, and more preferably 0.018 to 0.3 mass%.

### [C12]

The method according to any one of [A1] to [A18], [B1] to [B11], [C4] and [C8], wherein the additive is an Essential 8 medium, and the concentration (final concentration) of the Essential 8 medium in the serum-free medium is 0.5 to 100 mass%, preferably 0.5 to 50 mass%, and more preferably 0.625 to 30 mass%.

### [C13]

The method according to any one of [A1] to [A18], [B1] to [B11], [C5], [C6], and [C8], wherein the additive is recombinant human serum albumin, and the concentration (final concentration) of the recombinant human serum albumin in the serum-free medium is 0.001 to 1.0 mass%, preferably 0.01 to 0.5 mass%, and more preferably 0.015 to 0.3 mass%.

### [C14]

The method according to any one of [A1] to [A18], [B1] to [B11], [C5], [C7], and [C8], wherein the additive is non-human animal serum albumin, and the concentration (final concentration) of the non-human animal serum albumin in the serum-free medium is 0.01 to 2.0 mass%, preferably 0.05 to 1.5 mass%, and more preferably 0.125 to 0.75 mass%.

### [D1]

A method for producing a product using target cells, the method comprising producing target cells and producing a product using the target cells by culturing raw material cells according to the method according to any one of [A1] to [A18], [B1] to [B11] and [C1] to [C14].

### [D2]

The method according to [D1], wherein the raw material cells are megakaryocyte progenitor cells, the target cells are megakaryocytes, and the product is platelets.

### [D3]

The method according to [D1] or [D2], wherein the raw material cells are human megakaryocyte progenitor cells, the target cells are human megakaryocytes, and the product is human platelets.

### [D4]

The method according to [D1], wherein the raw material cells and the target cells are iPS cells, and the product is cytokines.

### [D5]

The method according to [D1] or [D4], wherein the raw material cells and the target cells are human iPS cells, and the product is human cytokines.

### [D6]

The method according to [D1], wherein the raw material cell and the target cell are mesenchymal stem cells, and the product is cytokines.

### [D7]

The method according to [D1] or [D6], wherein the raw material cells and the target cells are human mesenchymal stem cells, and the product is human cytokines.

### [E1]

A serum-free medium for use in production of target cells, the serum-free medium containing at least one culture factor selected from a growth factor and a differentiation inducing factor; human serum albumin; and at least one additive selected from a group consisting of a Src inhibitor, a PKC activator, methylcellulose, an Essential 8 medium, recombinant human serum albumin, and non-human animal serum albumin.

### [E2]

The serum-free medium according to [E1], wherein the target cells are megakaryocytes and the culture factor is a megakaryocyte differentiation inducing factor.

### [E3]

The serum-free medium according to [E1] or [E2], wherein the target cells are human megakaryocytes and the culture factor is a megakaryocyte differentiation inducing factor.

### [E4]

A serum-free medium for use in production of a product using target cells, the serum-free medium containing at least one culture factor selected from a growth factor and a differentiation inducing factor; human serum albumin; and at least one additive selected from a group consisting of a Src inhibitor, a PKC activator, methylcellulose, an Essential 8 medium, recombinant human serum albumin, and non-human animal serum albumin.

### [E5]

The serum-free medium according to [E4], wherein the production of the product using the target cells is production of platelets using megakaryocytes, and the culture factor is a megakaryocyte differentiation inducing factor.

### [E6]

The serum-free medium according to [E4] or [E5], wherein the production of the product using the target cells is production of human platelets using human megakaryocytes, and the culture factor is a megakaryocyte differentiation inducing factor.

### [E7]

The serum-free medium according to any one of [E1] to [E6], wherein the additive is a Src inhibitor, preferably A419259.

### [E8]

The serum-free medium according to any one of [E1] to [E6], wherein the additive is a PKC activator, preferably prostratin.

### [E9]

The serum-free medium according to any one of [E1] to [E6], wherein the additive is methylcellulose.

### [E10]

The serum-free medium according to any one of [E1] to [E6], wherein the additive is an Essential 8 medium.

### [E11]

The serum-free medium according to any one of [E1] to [E6], wherein the additive is recombinant human serum albumin or non-human animal serum albumin.

### [E12]

The serum-free medium according to any one of [E1] to [E6] and [E11], wherein the additive is recombinant human serum albumin, preferably recombinant human serum albumin expressed using rice or recombinant human serum albumin expressed using yeast.

### [E13]

The serum-free medium according to any one of [E1] to [E6] and [E11], wherein the additive is non-human animal serum albumin, preferably bovine serum albumin.

### [E14]

The serum-free medium according to any one of [E1] to [E13], wherein the concentration (final concentration) of the human serum albumin in the serum-free medium is 0.01 to 2.0 mass%, preferably 0.05 to 1.5 mass%, and more preferably 0.075 to 0.75 mass%.

### [E15]

The serum-free medium according to any one of [E1] to [E7] and [E14], wherein the additive is a Src inhibitor, preferably A419259, and the concentration (final concentration) of the Src inhibitor in the serum-free medium is 0.001 to 10 µM, preferably 0.01 to 1.0 µM, and more preferably 0.03 to 0.2 µM.

### [E16]

The serum-free medium according to any one of [E1] to [E6], [E8], and [E14], wherein the additive is a PKC activator, preferably prostratin, and the concentration (final concentration) of the PKC activator in the serum-free medium is 0.001 to 10 µM, preferably 0.01 to 1.0 µM, and more preferably 0.015 to 0.2 µM.

### [E17]

The serum-free medium according to any one of [E1] to [E6], [E9], and [E14], wherein the additive is methylcellulose, and the concentration (final concentration) of the methylcellulose in the serum-free medium is 0.001 to 3.0 mass%, preferably 0.01 to 1.0 mass%, and more preferably 0.018 to 0.3 mass%.

### [E18]

The serum-free medium according to any one of [E1] to [E6], [E10], and [E14], wherein the additive is an Essential 8 medium, and the concentration (final concentration) of the Essential 8 medium in the serum-free medium is 0.5 to 100 mass%, preferably 0.5 to 50 mass%, and more preferably 0.625 to 30 mass%.

### [E19]

The serum-free medium according to any one of [E1] to [E6], [E11], [E12], and [E14], wherein the additive is recombinant human serum albumin, and the concentration (final concentration) of the recombinant human serum albumin in the serum-free medium is 0.001 to 1.0 mass%, preferably 0.01 to 0.5 mass%, and more preferably 0.015 to 0.3 mass%.

### [E20]

The serum-free medium according to any one of [E1] to [E6], [E11], [E13], and [E14], wherein the additive is non-human animal serum albumin, and the concentration (final concentration) of the non-human animal serum albumin in the serum-free medium is 0.01 to 2.0 mass%, preferably 0.05 to 1.5 mass%, and more preferably 0.125 to 0.75 mass%.

### [Examples]

### [Example 1] Differentiation Culture from Megakaryocyte Progenitor Cells to Megakaryocytes

### (1) Method

In Example 1, a differentiation culture from human iPS cell-derived megakaryocyte progenitor cells to megakaryocytes was performed. The differentiation culture was performed using the method described in Article 2 (Ito et al., 2018, Cell 174, 636-648).

10U of heparin sodium (Yoshindo Inc., #(01) 14987476163428), 50 ng/ml of SCF (Stem Cell Factor) (Wako, #193-15513), 200 ng/ml of TA-316 (Nissan Chemical), 15 µM of KP-457 (Kaken Pharmaceutical), 0.5 µM of GNF-351 (Calbiochem), 0.5 µM of Y-39983 (MedChemExpress) were added to IMDM (Iscove's Modified Dulbecco's Medium) (Sigma-Aldrich, #I3390) (the concentrations were the final concentrations). A positive control medium was prepared by adding human plasma (HP) (Cosmo Bio) in an amount of 5 mass% to the resultant medium, and a medium to which human plasma was not added was used as a negative control (NC) medium.

In addition, media were prepared by adding various serum alternatives instead of human plasma. As the serum alternative, human serum albumin (HSA) (Sigma-Aldrich), rice-derived human recombinant albumin (R) (Sigma-Aldrich), yeast-derived human recombinant albumin (Y) (Wako), bovine serum albumin (BSA) (Sigma-Aldrich), an Essential 8 medium (E) (Invitrogen), an A419259 compound (A) (Wako), prostratin (P) (Wako), methylcellulose (M) (R & D Systems), or a combination thereof was used.

A differentiation culture of megakaryocytes was performed using these media. Specifically, the human iPS cell-derived megakaryocyte progenitor cells were cultured in a 96-well plate (Corning) with a medium volume of 100 µl per well for every 2 wells under each condition for 7 days.

After the differentiation culture, cells were analyzed using a confocal quantitative image cytometer CQ1 (Yokogawa Electric Corporation). The nuclei were stained with DRAQ5^{™} (Cosmo Bio), and the number of cells having a nuclear size of 20 µm or more (i.e., megakaryocytes) was counted in each well.

### (2) Results

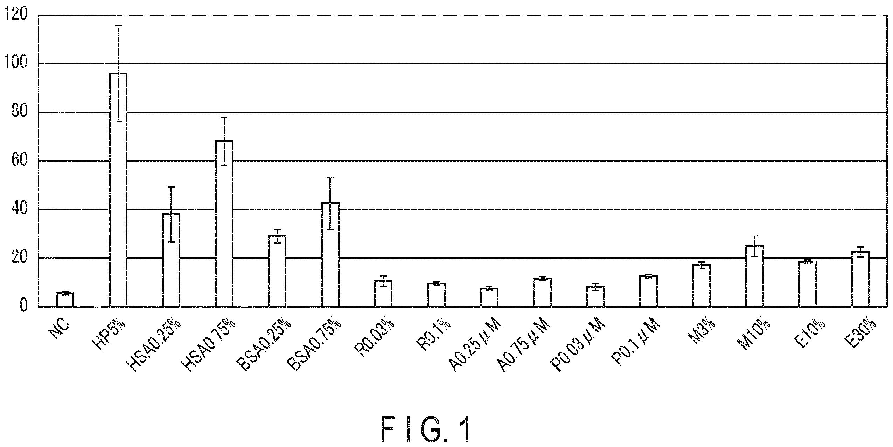

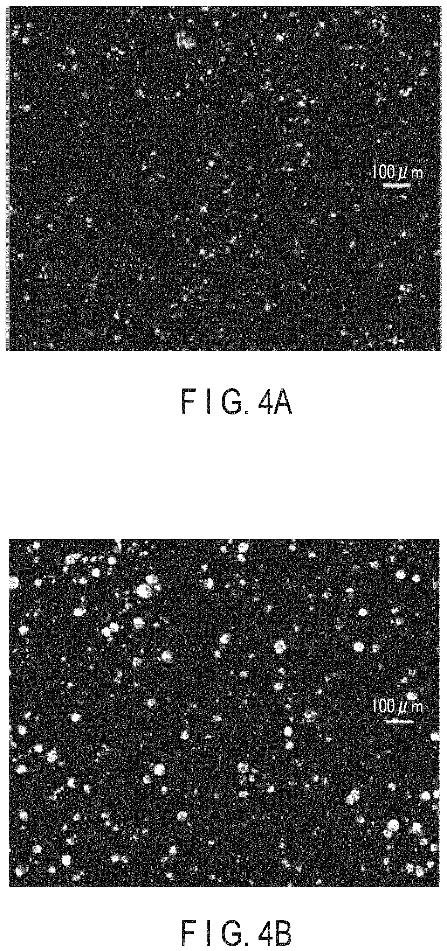

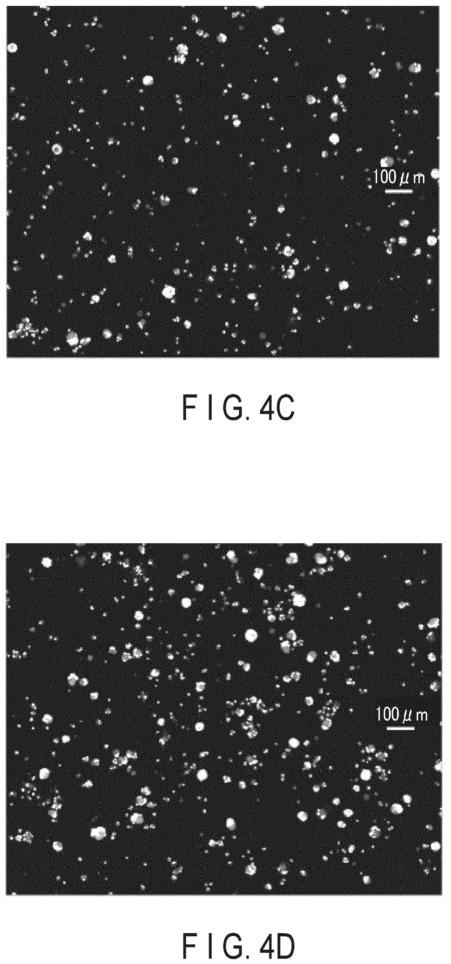

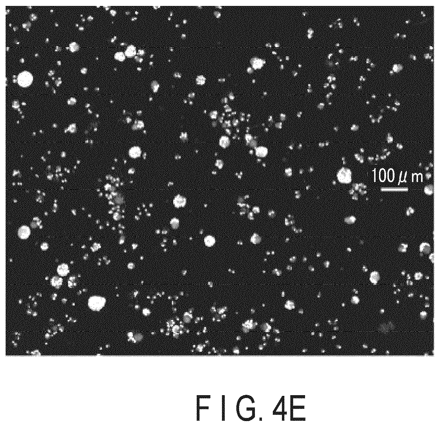

The results are shown in FIGS. 1, 2, 3, and 4A to 4E. FIGS. 1 to 3 are graphs showing the number of megakaryocytes differentiated in each medium. FIGS. 4A to 4E are photographs showing the megakaryocytes stained with a nuclear staining reagent (DRAQ5).

### (FIG. 1)

FIG. 1 shows the results of using the following media in order from the left.
Negative control: NC medium
Positive control: medium containing 5 mass% of human plasma (HP)
Example 1A: medium containing 0.25 mass% of human serum albumin (HSA)
Example 1B: medium containing 0.75 mass% of human serum albumin (HSA)
Example 1C: medium containing 0.25 mass% of bovine serum albumin (BSA)
Example 1D: medium containing 0.75 mass% of bovine serum albumin (BSA)
Example 1E: medium containing 0.03 mass% of rice-derived human recombinant albumin (R)
Example 1F: medium containing 0.1 mass% of rice-derived human recombinant albumin (R)
Example 1G: medium containing 0.25 µM of A419259 compound (A)
Example 1H: medium containing 0.75 µM of A419259 compound (A)
Example 1I: medium containing 0.03 µM of prostratin (P)
Example 1J: medium containing 0.1 µM of prostratin (P)
Example 1K: medium containing 3 mass% of 3% methylcellulose solution (M) (i.e., 0.09 mass% of medium containing methylcellulose)
Example 1L: medium containing 10 mass% of 3% methylcellulose solution (M) (i.e., 0.3 mass% of medium containing methylcellulose).
Example 1M: medium containing 10 mass% of Essential 8 medium
Example 1N: medium containing 30 mass% of Essential 8 medium
In Examples 1A to 1N, each of HSA, BSA, R, A, P, M, and E was added alone as the serum alternative. In all of Examples 1A to 1N, the number of megakaryocytes increased relative to the negative control (NC); however, there was a tendency for the number of megakaryocytes to be low relative to the positive control (HP 5%).

### (FIG. 2)

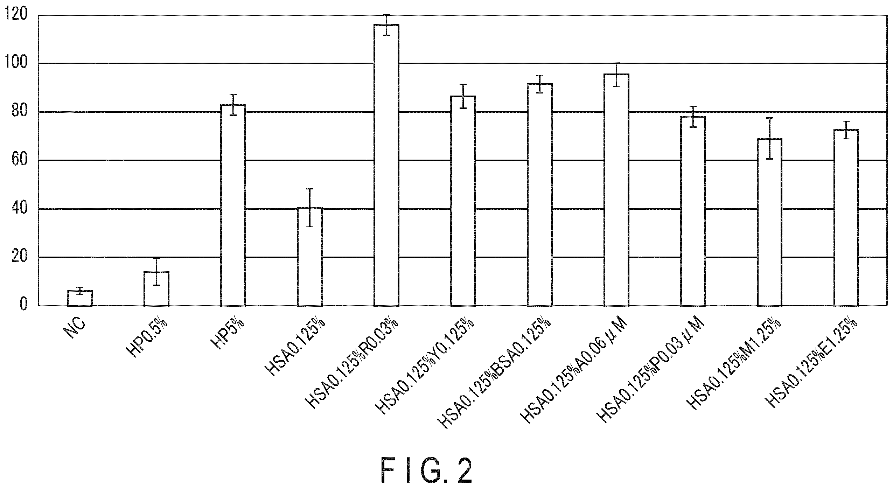

FIG. 2 shows the results of using the following media in order from the left.
Negative control: NC medium
Positive control 1: medium containing 0.5 mass% of human plasma (HP)
Positive control 2: medium containing 5 mass% of human plasma (HP)
Example 2A: medium containing 0.125 mass% of human serum albumin (HSA)
Example 2B: culture medium containing 0.125 mass% of human serum albumin (HSA) and 0.03 mass% of rice-derived human recombinant albumin (R)
Example 2C: medium containing 0.125 mass% of human serum albumin (HSA) and 0.125 mass% of yeast-derived human recombinant albumin (Y)
Example 2D: medium containing 0.125 mass% of human serum albumin (HSA) and 0.125 mass% of bovine serum albumin (BSA)
Example 2E: medium containing 0.125 mass% of human serum albumin (HSA) and 0.06 µM of A419259 compound (A)
Example 2F: medium containing 0.125 mass% of human serum albumin (HSA) and 0.03 µM of prostratin (P)
Example 2G: culture medium containing 0.125 mass% of human serum albumin (HSA) and 1.25 mass% of 3% methylcellulose (M) (i.e., 0.0375 mass% of methylcellulose).
Example 2H: culture medium containing 0.125 mass% of human serum albumin (HSA) and 1.25 mass% of Essential 8 medium
In Example 2A, 0.125 mass% of HSA was added alone as the serum alternative, and in Examples 2B to 2H, 0.125 mass% of HSA and each additive (any one of R, Y, BSA, A, P, M, and E) were added in combination as the serum alternative. In Examples 2B to 2H, there was a tendency for the number of megakaryocytes to increase compared to the case of HSA alone (Example 2A), and a number of megakaryocytes equivalent to that of the positive control 2 (HP 5%) was obtained.

### (FIG. 3)

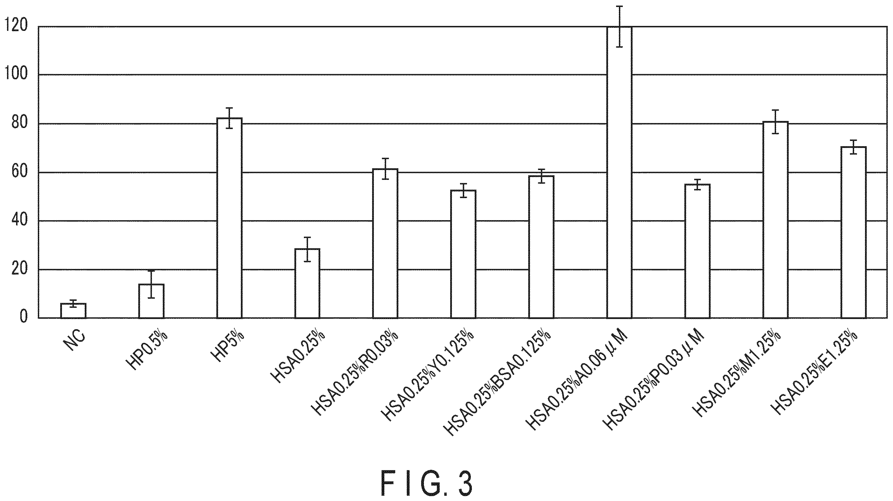

FIG. 3 shows the results of using the following media in order from the left.
Negative control: NC medium
Positive control 1: medium containing 0.5 mass% of human plasma (HP)
Positive control 2: medium containing 5 mass% of human plasma (HP)
Example 3A: medium containing 0.25 mass% of human serum albumin (HSA)
Example 3B: medium containing 0.25 mass% of human serum albumin (HSA) and 0.03 mass% of rice-derived human recombinant albumin (R)
Example 3C: medium containing 0.25 mass% of human serum albumin (HSA) and 0.125 mass% of yeast-derived human recombinant albumin (Y)
Example 3D: medium containing 0.25 mass% of human serum albumin (HSA) and 0.125 mass% of bovine serum albumin (BSA)
Example 3E: medium containing 0.25 mass% of human serum albumin (HSA) and 0.06 µM of A419259 compound (A)
Example 3F: medium containing 0.25 mass% of human serum albumin (HSA) and 0.03 µM of prostratin (P)
Example 3G: medium containing 0.25 mass% of human serum albumin (HSA) and 1.25 mass% of 3% methylcellulose (M) (i.e., 0.0375 mass% of methylcellulose).

Example 3H: medium containing 0.25 mass% of human serum albumin (HSA) and 1.25 mass% of Essential 8 medium.

In Example 3A, 0.25 mass% of HSA was added alone as the serum alternative, and in Examples 3B to 3H, 0.25 mass% of HSA and each additive (any one of R, Y, BSA, A, P, M, and E) were added in combination as the serum alternative. In Examples 3B to 3H, there was a tendency for the number of megakaryocytes to increase compared to the case of HSA alone (Example 3A), and a number of megakaryocytes equivalent to that of the positive control 2 (HP 5%) was obtained.

### (FIGS. 4A to 4E)

FIGS. 4A to 4E show DRAQ5 stained images of the negative control, the positive control 2, Example 2A, Example 2B, and Example 2E, respectively.

The number of megakaryocytes having large nuclei stained with DRAQ5 increases in Example 2B (HAS 0.125% + R 0.03%) and Example 2E (HAS 0.125% + A 0.06 µM) as compared to the negative control and Example 2A (HAS 0.125%), and this number is approximate to the number of cells in the positive control 2 (HP 5%).

### (3) Discussion

The results shown in FIGS. 1, 2, and 3, and 4A to 4E demonstrate that a differentiation culture of megakaryocyte progenitor cells using a combination of human serum albumin and a specific additive as a serum alternative enables production of megakaryocytes with a differentiation efficiency similar to that of the case of using human plasma. Since the serum alternative requires no blood component, the production cost of megakaryocytes can be reduced and the inter-lot stability of the megakaryocytes can be improved.

In addition, the inventors of the present invention demonstrated that the use of the media described below also allows the production of megakaryocytes with a differentiation efficiency similar to that of the case using human plasma.

A medium containing 0.5 mass% of human serum albumin (HSA) and 0.015 mass% of rice-derived human recombinant albumin (R)
A medium containing 0.5 mass% of human serum albumin (HSA) and 0.03 µM of A419259 compound (A)
A medium containing 0.5 mass% of human serum albumin (HSA) and 0.015 µM of prostratin (P)
A medium containing 0.5 mass% of human serum albumin (HSA) and 0.625 mass% of 3% methylcellulose solution (M) (i.e., 0.01875 mass% of methylcellulose)
A medium containing 0.5 mass% of human serum albumin (HSA) and 0.625 mass% of Essential 8 medium (E)
A medium containing 0.125 mass% of human serum albumin (HSA) and 0.015 mass% of rice-derived human recombinant albumin (R)
A medium containing 0.125 mass% of human serum albumin (HSA) and 0.03 µM of A419259 compound (A)
A medium containing 0.125 mass% of human serum albumin (HSA) and 0.015 µM of prostratin (P)
A medium containing 0.125 mass% of human serum albumin (HSA) and 0.625 mass% of 3% methylcellulose solution (M) (i.e., 0.01875 mass% of methylcellulose)
A medium containing 0.125 mass% of human serum albumin (HSA) and 0.625 mass% of Essential 8 medium

### [Example 2] Production of Platelets using Megakaryocytes

### (1) Method

By using the combination of human serum albumin (HSA) and rice-derived human recombinant albumin (R) and the combination of HSA and an A419259 compound (A), which were effective for megakaryocyte differentiation in Example 1, a shake flask culture was performed on a 25 ml scale in the same manner as in Example 1, thereby producing megakaryocyte-derived platelets.

After the culture, expression of platelet markers was analyzed using FACS (fluorescence activated cell sorting). The FACS analysis was performed using a flow cytometer (Sony SA3800).

Specifically, in order to analyze platelets, CD41 antibody-staining and CD42b antibody-staining were performed, thereby analyzing the ratio of CD41 positive cells and the ratio of CD42b positive cells in platelets. In order to examine the aggregation function of platelets, an increase in the ratio of PAC1 positive cells by the addition of 0.2 µM PMA (phorbol 12-myristate 13-acetate) and an increase in the ratio of PAC1 positive cells by the addition of 100 µM ADP (adenosine diphosphate) were analyzed.

### (2) Result

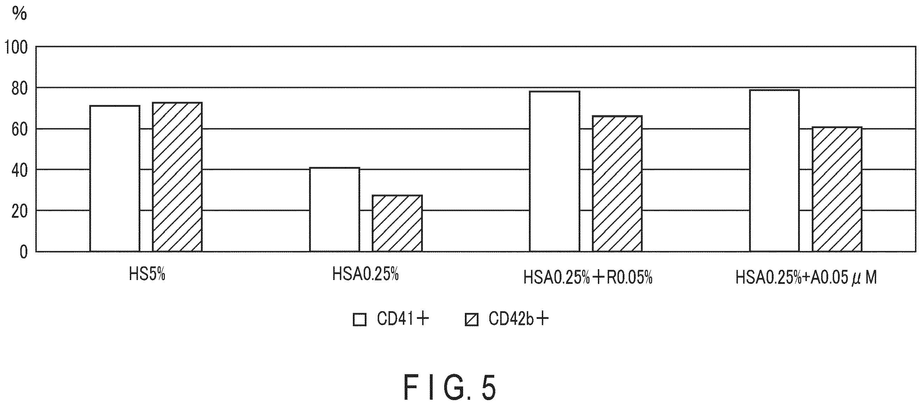

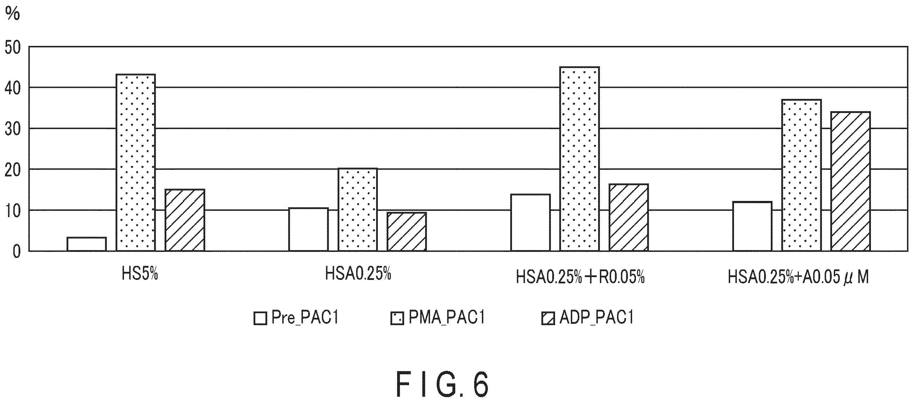

The results are shown in FIGS. 5 and 6.

### (FIG. 5)

FIG. 5 shows the ratio of positive cells in CD41 and CD42b. FIG. 5 shows the results of the case using the following media in order from the left.
Example 5A: medium containing 5 mass% of human plasma (HP)
Example 5B: medium containing 0.25 mass% of human serum albumin (HSA)
Example 5C: medium containing 0.25 mass% of human serum albumin (HSA) and 0.05 mass% of rice-derived human recombinant albumin (R)
Example 5D: medium containing 0.25 mass% of human serum albumin (HSA) and 0.05 µM of A419259 compound (A)
As compared to Example 5B (HAS 0.25%), Example 5C (HAS 0.25% + R 0.05%) and Example 5D (HAS 0.25% + A 0.05 µM) brought an increase in the ratio of CD41 positive cells and the ratio of CD42b positive cells, and achieved a ratio of CD41 positive cells and a ratio of CD42b positive cells equivalent to those of Example 5A (HP5%), which is the positive control.

### (FIG. 6)

FIG. 6 shows the ratio of PAC1 positive cells after PMA treatment and the ratio of PAC1 positive cells after ADP treatment. FIG. 6 shows the results of the case using the following media in order from the left.
Example 6A: medium containing 5 mass% of human plasma (HP)
Example 6B: medium containing 0.25 mass% of human serum albumin (HSA)
Example 6C: medium containing 0.25 mass% of human serum albumin (HSA) and 0.05 mass% of rice-derived human recombinant albumin (R)
Example 6D: medium containing 0.25 mass% of human serum albumin (HSA) and 0.05 µM of A419259 compound (A)
In FIG. 6, "Pre_PAC1" indicates the ratio of PAC1 positive cells before treatment with PMA or ADP, "PMA_PAC1" indicates the ratio of PAC1 positive cells after PMA treatment, and "ADP_PAC1" indicates the ratio of PAC1 positive cells after ADP treatment.

As compared to Example 6B (HAS 0.25%), Example 6C (HAS 0.25% + R 0.05%) and Example 6D (HAS 0.25% + A 0.05 µM) brought an increase in the ratio of PAC1 positive cells after PMA treatment and the ratio of PAC1 positive cells after ADP treatment, and achieved a ratio of PAC1 positive cells equivalent to that of Example 6A (HP 5%), which is the positive control.

### (3) Discussion

The results shown in FIGS. 5 and 6 demonstrate that a culture of megakaryocyte progenitor cells using the serum alternative (the combination of human serum albumin and a specific additive) which was effective for megakaryocyte differentiation in Example 1 enables production of functional platelets as in the case of using human plasma. Since the serum alternative requires no blood component, the production cost of platelets can be reduced and the lot stability of the platelets can be improved.

### [Example 3] Proliferation Culture of iPS Cells and Proliferation Culture of Mesenchymal Stem Cells

### (1) Method

The combination of human serum albumin (HSA) and recombinant human serum albumin and the combination of HSA and prostratin, which were effective in megakaryocyte differentiation in Example 1, were examined in terms of proliferation capacity of iPS cells. In addition, the combination of human serum albumin (HSA) and recombinant human serum albumin and the combination of HSA and an A419259 compound, which were effective in megakaryocyte differentiation in Example 1, were examined in terms of proliferation capacity of mesenchymal stem cells.

### (iPS Cells)

For iPS cells, iPS cells (253G1 strain) were seeded in 96 well plates (Corning) coated with iMatrix-511 (0.1 µg/well) (Nippi, Incorporated) for every 6 wells under each condition and at 1.0x10e4/well, and were cultured in the following media for 5 days.
Control: DMEM/F12 medium (Sigma-Aldrich)
Example 7A (HAS 0.125): DMEM/F12 medium containing 0.125 mass% of human serum albumin (HSA)
Example 7B (HAS 0.1 + R 0.025): DMEM/F12 medium containing 0.1 mass% of human serum albumin (HSA) and 0.025 mass% of rice-derived human recombinant albumin (R)
Example 7C (HAS 0.075 + Y 0.05): DMEM/F12 medium containing 0.075 mass% of human serum albumin (HSA) and 0.05 mass% of yeast-derived human recombinant albumin (Y)
Example 7D (HAS 0.125 + P 0.05): DMEM/F12 medium containing 0.125 mass% of human serum albumin (HSA) and 0.05 µM of prostratin (P)
Example 7E (R 0.025): DMEM/F12 medium containing 0.025 mass% of rice-derived human recombinant albumin (R)
Example 7F (Y 0.05): DMEM/F12 medium containing 0.05 mass% of yeast-derived human recombinant albumin (Y)
Example 7G (P 0.05): DMEM/F12 medium containing 0.05 µM of prostratin (P)
After the culture, the number of cells was measured by analyzing the fluorescence of DAPI-stained nuclei with a confocal quantitative image cytometer CQ1 (Yokogawa Electric Corporation).

### (Mesenchymal Stem Cells)

For mesenchymal stem cells, bone marrow-derived mesenchymal stem cells (JCRB1154 strain) were seeded in 96 well plates (Corning) for every 6 wells under each condition and at 1.0x10e4/well, and were cultured in the following media for 5 days.
Control: DMEM/F12 medium (Sigma-Aldrich)
Example 8A: (HAS 0.125): DMEM/F12 medium containing 0.125 mass% of human serum albumin (HSA)
Example 8B: (HAS 0.1 + R 0.025): DMEM/F12 medium containing 0.1 mass% of human serum albumin (HSA) and 0.025 mass% of rice-derived human recombinant albumin (R)
Example 8C: (HAS 0.075 + Y 0.05): DMEM/F12 medium containing 0.075 mass% of human serum albumin (HSA) and 0.05 mass% of yeast-derived human recombinant albumin (Y)
Example 8D (HAS 0.125 + A 0.05): DMEM/A419259 medium containing 0.125 mass% of human serum albumin (HSA) and 0.05 µM of F12 compound (A)
Example 8E: (R 0.025): DMEM/F12 medium containing 0.025 mass% of rice-derived human recombinant albumin (R)
Example 8F (Y 0.05): DMEM/F12 medium containing 0.05 mass% of yeast-derived human recombinant albumin (Y)
Example 8G (A 0.05): DMEM/F12 medium containing 0.05 µM of A419259 compound (A)
After the culture, the number of cells was measured by analyzing the fluorescence of DAPI-stained nuclei with a confocal quantitative image cytometer CQ1 (Yokogawa Electric Corporation).

### (2) Result

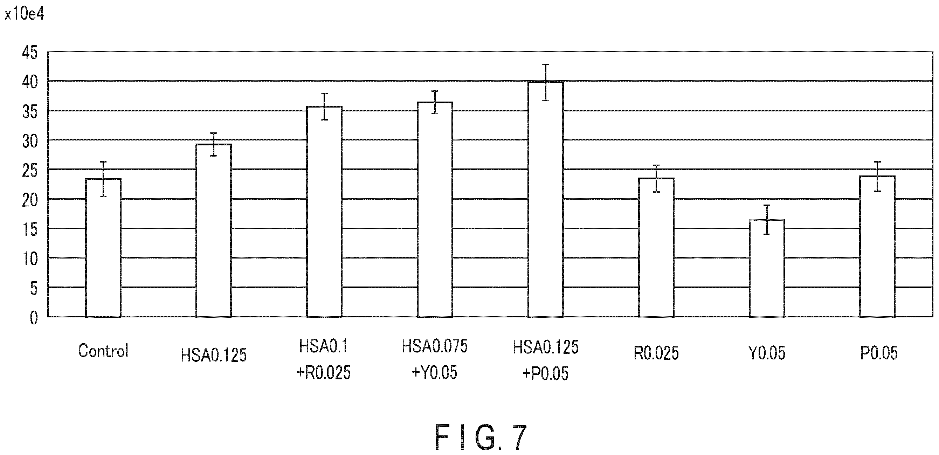

FIG. 7 shows the number of iPS cells after culture under each condition. As compared to the control and Example 7A (HSA alone), a significant increase in the number of iPS cells was observed in Examples 7B and 7C (a combination of HSA and recombinant human serum albumin) and Example 7D (a combination of HSA and prostratin) . On the other hand, in Example 7E and Example 7F (recombinant human serum albumin alone) and Example 7 (prostratin alone), no significant increase in the number of iPS cells was observed.

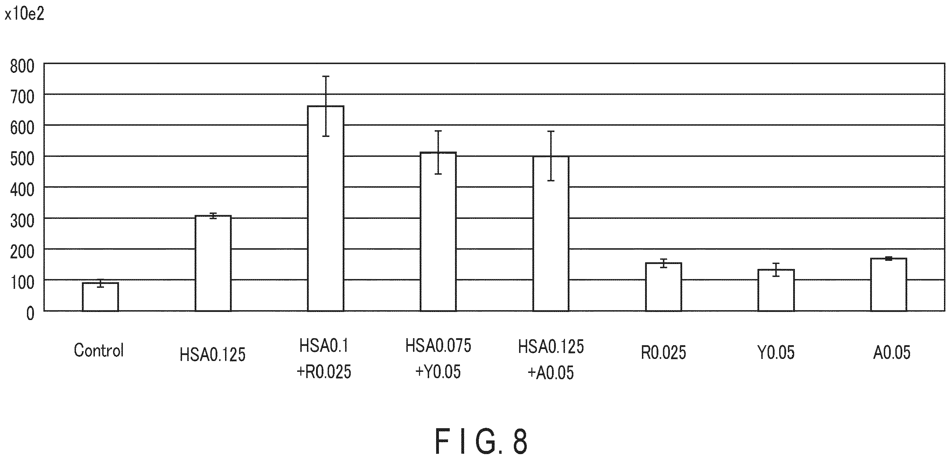

FIG. 8 shows the number of MSC cells after culture under each condition. As compared to the control and Example 8A (HSA alone), a significant increase in the number of MSC cells was observed in Example 8B and 8C (a combination of HSA and recombinant human serum albumin) and Example 8D (a combination of HSA and A419259). On the other hand, no significant increase in the number of MSC cells was observed in Example 8E and Example 8F (recombinant human serum albumin alone) and Example 8G (A419259 alone).

### (3) Discussion

The results shown in FIGS. 7 and 8 demonstrate that a proliferation culture of iPS cells and a proliferation culture of mesenchymal stem cells using the serum alternative (a combination of human serum albumin and a specific additive) which was effective for megakaryocyte differentiation in Example 1 achieves high proliferation efficiency because of a synergistic effect. Since the serum alternative requires no blood component, the production cost of cells by the proliferation culture can be reduced and the lot stability of the proliferating cells can be improved.

## Claims

1. A method for producing a target cell, the method comprising producing the target cell by culturing a raw material cell in a serum-free medium containing:
at least one culture factor selected from a growth factor and a differentiation inducing factor;
human serum albumin; and
at least one additive selected from a group consisting of a Src inhibitor, a PKC activator, methylcellulose, an Essential 8 medium, recombinant human serum albumin, and non-human animal serum albumin.

2. The method according to claim 1, wherein the culture factor is at least one differentiation inducing factor and the target cell is a differentiated cell.

3. The method according to claim 2, wherein the raw material cell is selected from a group consisting of a megakaryocyte progenitor cell, an iPS cell, a mesenchymal stem cell, and a fibroblast.

4. The method according to claim 2 or 3, wherein the target cell is selected from a group consisting of a megakaryocyte, a mesenchymal stem cell, a fibroblast, and a cardiomyocyte.

5. The method according to any one of claims 2 to 4, wherein the raw material cell is a megakaryocyte progenitor cell and the target cell is a megakaryocyte.

6. The method according to claim 1, wherein the culture factor is at least one growth factor and the target cell is a proliferating cell.

7. The method according to claim 6, wherein the raw material cell and the target cell are selected from a group consisting of an iPS cell and a mesenchymal stem cell.

8. The method according to any one of claims 1 to 7, wherein the additive is A419259.

9. The method according to any one of claims 1 to 7, wherein the additive is prostratin.

10. The method according to any one of claims 1 to 7, wherein the additive is methylcellulose.

11. The method according to any one of claims 1 to 7, wherein the additive is an Essential 8 medium.

12. The method according to any one of claims 1 to 7, wherein the additive is recombinant human serum albumin or non-human animal serum albumin.

13. A method for producing a product using a target cell, the method comprising producing the target cell by culturing a raw material cell according to the method according to any one of claims 1 to 12 and producing the product using the target cell.

14. The method according to claim 13, wherein the raw material cell is a megakaryocyte progenitor cell, the target cell is a megakaryocyte, and the product is a platelet.

15. A serum-free medium for use in production of a target cell, the serum-free medium comprising:
at least one culture factor selected from a growth factor and a differentiation inducing factor;
human serum albumin; and
at least one additive selected from a group consisting of a Src inhibitor, a PKC activator, methylcellulose, an Essential 8 medium, recombinant human serum albumin, and non-human animal serum albumin.

16. The serum-free medium according to claim 15, wherein the target cell is a megakaryocyte and the culture factor is a megakaryocyted differentiation inducing factor.

17. A serum-free medium for use in production of a product using a target cell, the serum-free medium comprising:
at least one culture factor selected from a growth factor and a differentiation inducing factor;
human serum albumin; and
at least one additive selected from a group consisting of a Src inhibitor, a PKC activator, methylcellulose, an Essential 8 medium, recombinant human serum albumin, and non-human animal serum albumin.

18. The serum-free medium according to claim 17, wherein the production of the product using the target cell is production of a platelet using a megakaryocyte, and the culture factor is a megakaryocyte differentiation inducing factor.
